# EUROPÄISCHE PATENTSCHRIFT

(11) **EP 2 118 714 B1**
(45) Veröffentlichungstag und Bekanntmachung des Hinweises auf die Patenterteilung: **17.07.2013**
(21) Anmeldenummer: 07866273.1
(22) Anmeldetag: 28.12.2007
(51) Int. Cl.: G05D 11/13, A61B 1/00, A61H 1/00, A63B 22/02

(54) **VORRICHTUNG ZUR ÜBERWACHUNG, STEUERUNG UND/ODER REGELUNG EINER GASZUSAMMENSETZUNG**
DEVICE FOR MONITORING, CONTROLLING AND/OR REGULATING A GAS COMPOSITION
DISPOSITIF DE CONTRÔLE, DE COMMANDE ET/OU DE RÉGULATION D'UNE COMPOSITION DE GAZ

(30) Priorität: 12.01.2007 DE 102007002614
(43) Veröffentlichungstag der Anmeldung: 18.11.2009
(73) Patentinhaber: Jerichow, Ulrich, 63571 Gelnhausen (DE)
(72) Erfinder: Jerichow, Ulrich, 63571 Gelnhausen (DE)
(74) Vertreter: Metten, Karl-Heinz
(86) Internationale Anmeldenummer: PCT/EP2007/011443
(87) Internationale Veröffentlichungsnummer: WO 2008/083842

(56) Entgegenhaltungen:
- EP-A- 0 176 277
- WO-A-03/024505
- DE-U1-202006 011 058

## Beschreibung

Die vorliegende Erfindung betrifft eine Vorrichtung zur Überwachung, Steuerung und/oder Regelung einer Gaszusammensetzung in einer Trainings- und/oder Rehabilitationsanlage.

Es ist bekannt, ein Höhentraining zur Steigerung der konditionellen Leistungsfähigkeit, beispielsweise eines Sportlers, durchzuführen. Dabei wird ein entsprechender Trainingseffekt durch längere Trainingsaufenthalte in Hochgebirgsregionen erreicht. Eine exakt steuerbare und kontrollierte Leistungssteigerung ist bei einem solchen Höhentraining allerdings nicht möglich.

Für eine genaue Untersuchung der Lungenfunktion kann das Verfahren der Spirometrie angewendet werden. So wird beispielsweise in der DE 6 912 241 U eine Spirometrie-Atemmaske zur Untersuchung der Lungenfunktion, insbesondere bei körperlicher Belastung, beschrieben. Weiterhin kann gemäß der US 2006/0 201 507 A1 eine Spirometrie-Vorrichtung für die Messung der Sauerstoffaufnahme genutzt werden. Auch kann gemäß Dokument EP 0 176 277 A in eine Vorrichtung die eine Trainingseinheit umfasst die Trainingsschwere durch die Atemflusszusammensetzung beeinflusst werden. Mit solchen Systemen ist es allerdings nicht möglich, gleichzeitig die Sauerstoff- und Kohlendioxidzusammensetzung der Inspirationsluft sowie der Expirationsluft zu überwachen.

Um das Ausdauertraining bei gleichzeitiger Überwachung der Leistungsdaten sowie der Körperfunktionen für einen Sportler angenehmer zu gestalten, ist eine Integration der Trainingseinrichtung in ein System zur Bereitstellung einer virtuellen Realität möglich. Gemäß der DE 20 2004 007 273 U1 ist bereits eine Erholungs- und/oder Erlebniseinrichtung, insbesondere Wellness-Anlage, mit Filmpräsentation mittels einer Filmvorführeinrichtung und einem Filmabbildungsbereicht innerhalb geschlossener Räume beschrieben, wobei diese über eine Steuereinrichtung miteinander verbundene Bewegungs-, Ton-, Wind-, Geruchs-, Licht- und/oder sonstige Strahlungs-Erzeugungseinrichtungen und/oder Sensoreinrichtungen enthält, deren Aktivitäten und Funktionsweisen auf Ereignisse der Filmpräsentation zeitlich, örtlich und/oder in ihren Intensitäten abgestimmt sind. Dabei erfolgt bei solchen und ähnlichen Systemen aber keine Aufnahme und Analyse der Körperfunktionen, wie der Lungenfunktion, so dass auch keine Kontrolle über die Wirkung oder den Erfolg der Anwendung möglich ist. Weiterhin können mit solchen Systemen keine speziellen Fitness- bzw. Trainingsprogramme und auch keine medizinischen Anwendungen durchgeführt werden.

Die Aufgabe der vorliegenden Erfindung besteht darin, ein System zur Verfügung zu stellen, mit dem eine Person beispielsweise spezielle Fitness- oder Rehabilitationsprogramme absolvieren kann, wobei gleichzeitig insbesondere die Sauerstoff- und die Kohlendioxidzusammensetzung der Inspirationsluft sowie der Expirationsluft überwacht, der Sauerstoffgehalt der Inspirationsluft je nach Bedarf verändert sowie das Atemflussvolumen gemessen werden können.

Die Aufgabe wurde durch eine Vorrichtung zur Überwachung, Steuerung und/oder Regelung einer Gaszusammensetzung gelöst, wobei die Vorrichtung mindestens
- eine Trainings und/oder Rehabilitationseinheit,
- eine Sensor-Einheit mit einem beheizbaren elektrochemischen Festelektrolyt-Sensor zur Sauerstoffkonzentrationsbestimmung und einem weiteren beheizbaren elektrochemischen Festelektrolyt-Sensor für die Kohlendioxid-Konzentratiönsbestimmung,
- eine Steuereinheit für die Sensoren,
- einen Micro-Controller in der Steuereinheit für die vom Atemflussvolumen abhängige Steuerung der Heizkraft von Heizelementen der Sensoren zur Aufrechterhaltung konstanter Sensor-Temperaturen sowie
- ein System zur Ab- und/oder Anreicherung von Luftbestandteilen zur Erzeugung der Gaszusammensetzung unter Vorgabe von bestimmbaren Sollwerten und/oder in Abhängigkeit der von der Sensor-Einheit ermittelten Atemgaszusammensetzung sowie des ermittelten Atemflussvolumens
umfasst.

Ein entscheidender Vorteil einer solchen Vorrichtung ergibt sich durch die Möglichkeit, die Sauerstoff- und/oder Kohlendioxidkonzentration sowie das Atemflussvolumen jeweils getrennt bzw. zuordenbar für die Inspirations- und auch die Expirationsluft bestimmen zu können. Ein weiterer Vorteil ergibt sich dadurch, dass bei einem Trainingsvorgang oder auch im Ruhezustand die Lungenfunktion einer die Vorrichtung nutzenden Person exakt überwacht werden kann. Weiterhin kann beispielsweise eine Unterversorgung mit Sauerstoff sofort erkannt werden und das Training entsprechend modifiziert oder abgebrochen werden. Außerdem ist ein aufwendiges Kalibrieren, wie es bei Spirometrie-Vorrichtungen erforderlich ist, nicht mehr notwendig.

Bei einer vorteilhaften Ausführungsvariante ist die Sensoreinheit unmittelbar in einem von der Inspirations- und Expirationsluft der Person durchströmten Bauteil angeordnet. So kann die Sensoreinheit beispielsweise in einer Atemmaske, welche von einer Person getragen wird, eingebaut sein. Diese Anordnung hat den besonderen Vorteil, dass ein extrem geringes Totvolumen vorhanden ist.

Im Sinne dieser Erfindung kann die das System zur Ab- und/oder Anreicherung von Luftbestandtellen verlassende an bzw. abgereicherte Gaszusammensetzung über Schläuche der Atemmaske zugeführt werden und somit als Inspirationsluft dienen oder der Inspirationsluft beigemischt werden.

Weiterhin ist es von Vorteil, dass der Sauerstoffsensor zur selektiven Leitung von Sauerstoff-Ionen Yttrium-dotiertes Zirkonoxid als Elektrolyt zwischen zwei Elektroden sowie ein Trägerelement und ein Heizelement enthält und der Kohlendioxidsensor einen Elektrolyt aus einem superschnellen Natrium-Ionenleiter, zwei Elektroden, ein Trägerelement und ein Heizelement enthält (1). Der genannte superschnelle Natrium-Ionenleiter, auch NASICON genannt, kann durch die Formel Na₃₋ₓZr₂(PO₄)₁₊ₓ(SiO₄)₂₋ₓ) beschrieben werden (2). Sensoren dieser Art haben den Vorteil, dass sie besonders klein und leicht sowie kostengünstig hergestellt werden können. So können für derartige Sensoren beispielsweise Abmaße von 20 x 3,5 x 0,5 mm erreicht werden (1). Solche miniaturisierten Sensoren sind damit für einen Einbau in eine Atemmaske besonders geeignet.

Für eine Messung der Sauerstoff-Sättigung des Blutes ist es von Vorteil, wenn ein Ohr-Clip in die Vorrichtung integriert ist. Außerdem kann die Vorrichtung ein Ohr-Clip für eine Messung des Pulses des Benutzers enthalten. Mit der Vorrichtung können somit umfangreiche Leistungsdaten erfasst sowie weitere medizinische Kenngrößen des Nutzers, wie beispielsweise der Herzfrequenz, aufgezeichnet werden. Die erhaltenen Messdaten können in vorteilhafter Weise mit Hilfe eines angeschlossenen Personal Digital Assistant (PDA) aufgezeichnet werden.

Die Trainings- und/oder Rehabilitationseinheit kann beispielsweise ein Rudergerät, Fahrrad oder Laufband sein.

Bei vorteilhaften Ausgestaltungen des Systems zur Ab- und/oder Anreicherung von Luftbestandteilen können ein oder mehrere Filter zur An- oder Abreicherung von Gasbestandteilen, vorzugsweise zur Abreicherung von Sauerstoff, enthalten sein. So kann die Möglichkeit zur Abreicherung von Sauerstoff besonders vorteilhaft für eine Simulation eines Höhentrainings verwendet werden. Selbstverstandlich kann das System zur Ab- und/oder Abreicherung von Luftbestandteilen beliebig gestaltet sein. So kann beispielsweise ein Sauerstoffkonzentrator mit einem Membranfilter vorhanden sein, wobei je nach Bedarf die das System verlassende Gaszusammensetzung mit angereichertem oder abgereichertem Sauerstoff genutzt werden kann.

Weiterhin kann die Vorrichtung bei vorteilhafter Ausgestaltung Mittel zur zwei- und dreidimensionalen visuellen Darstellung, mindestens ein akustisches Ausgabe- und/oder Aufnahmemittel und Mittel zur Erzeugung von Wind- Temperatur und/oder Geruch umfassen. Weiterhin kann die Vorrichtung ein Mittel für die Stimulation des Tastsinns und/oder ein Mittel zur Veränderung der Zusammensetzung der Atemluft enthalten. Ferner ist es von Vorteil, dass die Komponenten der Trainings- und/oder Rehabilitationseinheit, des Systems zur Ab und/oder Anreicherung von Luftbestandteilen, der Sensor-Einheit und der Steuereinheit für die Sensoren über ein Computersystem miteinander verbunden sind sowie über ein solches Computersystem gesteuert und/oder ausgelesen werden. Dabei kann das Computersystem mindestens aus einem Steuercomputer mit einer Benutzeroberfläche bestehen.

Bei einer vorteilhaften Ausführungsvariante der Vorrichtung sind an den Steuercomputer über ein Netzwerkrechner zur Bildberechnung für das rechte und linke Auge angeschlossen. Die dabei generierten Signale können an einen auf dem Kopf des Nutzers getragenen Helm mit LCDs zur Erzeugung einer virtuellen Umgebung (Head Mounted Display HMD) weitergeleitet werden. Alternativ können die generierten Signale auch für eine Stereoproduktion zur Erzeugung einer dreidimensionalen Darstellung auf einer Leinwand genutzt werden. Vorteilhaft ist es weiterhin, wenn der Steuercomputer mit einem oder mehreren Eingabegeräten mit mindestens sechs Freiheitsgraden zur Bestimmung der Position und Orientierung verbunden ist und die Eingabegeräte wahlweise mit einer oder mehreren Tasten ausgestattet sind. Vorteilhaft ist es ferner, dass beispielsweise isometrische, isotone und/oder elastische Eingabegeräte an den Steuercomputer angeschlossen sind, wobei mit diesen Eingabegeräten beispielsweise eine Blickbewegungserfassung, Körperbewegungserfassung, Kopfbewegungserfassung und/oder Positionsbestimmung erfolgen kann. Bei einer weiteren vorteilhaften Ausgestaltung kann mit den Eingabegeräten Gestik, Mimik und/oder Sprache erfasst werden. Somit wird eine Kombination aus physischen und psychischen Reizen ermöglicht und eine Aromaanwendung oder ein Höhentraining in einem virtuellen dreidimensionalen Umfeld durchführbar.

In einer besonders vorteilhaften Ausführungsvariante wird als Eingabegerät beispielsweise ein Head Traker verwendet, der auch an dem auf dem Kopf des Nutzers getragenen Helm mit LCDs zur Erzeugung der virtuellen Umgebung (Head Mounted Display HMD) befestigt sein kann. Vorteilhaft ist es ferner, dass die visuelle Darstellungseinheit ein nicht bewegtes Bild, einen bewegten oder nicht bewegten Gegenstand, eine Computergrafik und/oder zwei- und/oder dreidimensional bewegte Bilder oder Filme wiedergibt. Es können dazu auch konventionelle Monitore für die zweidimensionale Darstellung verwendet werden.

Bei einer vorteilhaften Ausgestaltung kann die visuelle Darstellungseinheit ein Bild mit einem Sichtwinkel von 0 bis 179° wiedergeben oder für die Verwendung des Systems in den Bereichen Fitness, Wellness oder Medizin auch ein Bild mit einem Sichtwinkel von 180° oder mehr als 180° wiedergeben, wobei auch vom Benutzer vorher aufgenommene bewegte und/oder unbewegte reale Bilder dargestellt werden können.

Die akustische Ausgabeeinheit kann beispielsweise Musikinstrumente, menschliche Stimmen, Umgebungsgeräusche, wie Tierlaute, Wind, Regen, Wasserfälle, Donner und/oder Geräusche von Fahrzeugmotoren, Schüssen, Pumpen, Explosionen und/oder Erdarbeiten wiedergeben. Besonders vorteilhaft ist es, wenn Wind, Temperatur, Geruch und/oder Luftfeuchtigkeit an die dagestellte Situation in der virtuellen Realität angepasst werden können.

Weiterhin ist es von Vorteil, wenn über eine Kommunikationseinheit Anweisungen und/oder Hinweise an den Benutzer der Vorrichtung gegeben werden können und der Benutzer über eine Kommunikationseinheit mit einer die Vorrichtung beginnenden Person in Kontakt treten kann. Bei einer vorteilhaften Weiterentwicklung des Systems können durch die Entnahme von Blut auch genauere Blutbildanalysen vor, während und/oder nach der Benutzung durchgeführt werden. Beispielsweise kann mit Hilfe eines mit dem Computersystem verbundenen Zellanalysegeräts, vorzugsweise eines Geräts für die Durchflusszytometrie, die Zusammensetzung der Blutzellen exakt bestimmt werden. Auch ist unter Verwendung, vorzugsweise mit einem Fluoreszenzfarbstoff gekoppelten, spezifischen Antikörpers eine Analyse von Oberflächenmarkern auf Zellen möglich.

Im Sinne dieser Erfindung ist weiterhin Verfahren zur gleichzeitigen Überwachung, Steuerung und/oder Regelung einer Gaszusammensetzung, wobei
- eine Person eine Trainingseinheit und/oder eine Rehabilitationseinheit benutzt,
- in einer Sensor-Einheit eine Sauerstoffkonzentrationsbestimmung mit Hilfe eines beheizbaren elektrochemischen Festelektrolyt-Sensors und eine Kohlendioxid-Konzentrationsbestimmung mit Hilfe eines weiteren beheizbaren elektrochemischen Festelektrolyt-Sensors erfolgt,
- eine vom Atemflussvolumen der Person abhängige Steuerung der Heizkraft von Heizelementen der Sensoren zur Aufrechterhaltung konstanter Sensor-Temperaturen mit Hilfe eines Micro-Controllers in einer Sensor-Steuereinheit erfolgt sowie
- unter Vorgabe von bestimmbaren Sollwerten und/oder in Abhängigkeit der von der Sensor-Einheit ermittelten Atemgaszusammensetzung sowie des ermittelten Atemflussvolumens der Personen ein System zur Erzeugung der Gaszusammensetzung Luftbestandteile ab- und/oder anreichert.

Dieses erfindungsgemäße Verfahren kann unter Verwendung der oben beschriebenen Vorrichtung in einer oder mehreren der genannten Ausführungsformen durchgeführt werden. Besonders vorteilhaft erfolgt die Bestimmung der Sauerstoffkonzentration der Atemluft durch Messung des bei konstanter Spannung durch den Elektrolyt des Sauerstoffsensors von der Kathode zur Anode fließenden Stroms, wobei ein linearer Zusammenhang zwischen dem resultierenden elektrischen Strom und der Sauerstoffkonzentration besteht. Ferner ist es von Vorteil, wenn die Kohlendioxidkonzentration über einen logarithmischen Zusammenhang zwischen der Spannung zwischen den Elektroden des Kohlendioxidsensors und der Kohlendioxidkonzentration bestimmt wird. Ferner ist es vorteilhaft, dass das Atemflussvolumen aus der durch den Mikro-Controller gesteuerten - zur Aufrechterhaltung einer konstanten Sensortemperatur notwendigen - Heizkraft der Heizelemente der Sensoren bestimmt wird.

Die Bestimmung der Gesamtflussrate der Atemluft kann mit dem Sensorelement unter Ausnutzung der Dünnschicht Anemometrie erfolgen. Weiterhin kann die Flussrichtung des Atemgases entweder durch Verwendung der gemessenen Sauerstoff- und/oder Kohlendioxidkonzentrationsgradienten oder des Temperaturprofils auf dem Sensor ermittelt werden. Das erfindungsgemäße Verfahren hat den Vorteil, dass gleichzeitig der Volumenstrom, die Strömungsrichtung und somit die Sauerstoff- und Kohlendioxidzusammensetzung der Inspirationsluft sowie der Expirationsluft mit einer Atemzug-für-Atemzug Auflösung überwacht werden können. Die Sauerstoff- und Kohlendioxid-Konzentrationen können also der Inspirationsluft und der Expirationsluft eindeutig zugeordnet werden. Besonders vorteilhaft ist es dabei, wenn der Sauerstoffgehalt der Inspirationsluft je nach Bedarf verändert wird.

Weiterhin ist es von Vorteil, wenn eine Unterversorgung mit Sauerstoff an der Sauerstoffkonzentration der Expirationsluft festgestellt wird. Bei einer vorteilhaften Ausgestaltung des erfindungsgemäßen Verfahrens wird bei einer Unterversorgung der Sauerstoffgehalt der Inspirationsluft erhöht oder das Programm abgebrochen. Gemäß dem erfindungsgemäßen Verfahren kann der Nutzer den Sauerstoffgehalt innerhalb von wenigen Sekunden von einem niedrigen Wert zwischen 9 Vol.-% bis zu einem hohen Wert von bis zu 100 Vol.-% und umgekehrt verändern, wobei besonders vorzugsweise eine Anpassung in einem Bereicht von 16 Vol.-% bis zu 21 Vol.-% erfolgen kann. Somit kann die erfindungsgemäße Vorrichtung zur Steigerung der Ausdauerleistung, vorzugsweise mittels eines simulierten Höhentrainings, verwendet werden. Bei dieser Verwendung ist es besonders vorteilhaft, dass die Sauerstoffkonzentration innerhalb von wenigen Sekunden von einem niedrigen Wert zwischen 9 Vol.-% bis zu einem hohen Wert von bis zu 100 Vol.-% und umgekehrt verändert werden kann, wobei eine Anpassung besonders vorzugsweise in einem Bereich von 16 Vol.-% bis zu 21 Vol.-% erfolgt.

Es ist ferner von Vorteil, dass ein Computerprogramm mit Programmcode zur Durchführung aller oben genannten erfindungsgemäßen Verfahrensschritte genutzt wird, wenn das Programm in einem Computer ausgeführt wird. Dabei ist es von Vorteil, wenn das Computerprogramm mit Programmcode zur Durchführung aller oben genannten Verfahrensschritte auf einem maschineniesbaren Träger gespeichert ist, wenn das Programm in einem Computer ausgeführt wird.

Unter Anwendung der erfindunsgemäßen Vorrichtung und/oder des erfindunsgemäßen Verfahrens können sich beispielsweise Spitzen- und Leistungssportler optimal mit Höhentrainingseinheiten im virtuellen realitätsnahen Umfeld auf anstehende Wettkämpfe vorbereiten. Das realitätsnahe Training unter sauerstoffarmen Bedingungen zielt bei Breiten- und Freizeitsportlern eher auf die Steigerung der persönlichen Leistungsfähigkeit und des individuellen Konditionsniveaus ab. Dabei können im speziellen die kosten- und zeitintensiven Flüge und Aufenthalte in Hochgebirgsregionen eingespart werden. Weiterhin ist ein wesentlich effizienteres Training möglich, da die Anlage 24 Stunden verfügbar und logistisch leicht erreichbar ist.

Im Bereich Rehabilitation oder Wellness könnte dieses System in einem virtuellen dreidimensionalen Umfeld beispielsweise eine Aromaanwendung mit einem passiven Höhentraining und einer Sauerstofftherapie kombinieren. In einer solchen Umgebung könnte eine solche Kombination aus Entspannung und Verbesserung der persönlichen Leistungsfähigkeit sowie eine Stärkung des Immunsystems erreicht werden.

Im Bereich der Medizin kann das System für eine Aromaanwendung, ein Höhentraining und/oder eine Sauerstofftherapie in einem dreidimensionalen Umfeld genutzt werden, wobei die vier Sinne Sehen, Fühlen, Riechen und Hören stimuliert werden. Durch die dabei erreichte Mobilisierung des körpereigenen Abwehrsystems ist eine Anwendung bei Personen mit Erkrankungen wie beispielsweise Krebs, Allergien und Erkrankungen des Stoffwechsels denkbar.

Weiterhin bietet besonders die Technik der dreidimensionalen Darstellung die Möglichkeit, spezielle psychische Erkrankungsverläufe, wie Ängste bei Autoimmunsystemerkrankungen, durch die Wirkung von Bildern und Geräuschen positiv zu beeinflussen.

### Literatur:

(1) R. Baumann^{1,2}, S. Fasoulas¹, M. Gläser¹, C. Gritzner¹, F. Hammer², J. , Heisig¹, R. Kahle¹, T. Kirschke¹, T. Schmiel¹, M. Völkel². Solid State Electrolyte Sensors for the Determination of Oxygen, Carbon Dioxide, and Total Flow Rates Associated to Respiration in Human Subjects. Institute for Aerospace Engineering, Technische Universität Dresden, 01062 Dresden, Germany ²ESCUBE GmbH, Nobelstr. 15, 70569 Stuttgart, Germany (PRO2-FR-Exec-Sum-05-02-10 Executive Summary to the ESTEC Contract No. 15450/01/NUJS CCN 1+2)
(2) West, A. R., Grundlagen der Festkörperchemie, Verlag Chemie, Weinheim (1992).

## Patentansprüche

1. Vorrichtung zur Überwachung, Steuerung und/oder Regelung einer Gaszusammensetzung, **dadurch gekennzeichnet, dass** die Vorrichtung mindestens
- eine Trainings- und /oder Rehabilitationseinheit,
- eine Sensor-Einheit mit einem beheizbaren elektrochemischen Festelektrolyt-Sensor zur Sauerstoffkonzentrationsbestimmung und einem weiteren beheizbaren elektrochemischen Festelektrolyt-Sensor für die Kohlendioxid-Konzentrationsbestimmung,
- eine Steuereinheit für die Sensoren,
- einen Micro-Controller in der Steuereinheit für die vom Atemflussvolumen abhängige Steuerung der Heizkraft von Heizelementen der Sensoren zur Aufrechterhaltung konstanter Sensor-Temperaturen sowie
- ein System zur Ab- und/oder Anreicherung von Luftbestandteilen zur Erzeugung der Gaszusammensetzung unter Vorgabe von bestimmbaren Sollwerten und/oder in Abhängigkeit der von der Sensor-Einheit ermittelten Atemgaszusammensetzungen sowie des ermittelten Atemflussvolumens
umfasst.

2. Vorrichtung nach Anspruch 1, **dadurch gekennzeichnet, dass** die Sensor-Einheit unmittelbar in einem von der Inspirations- und Expirationsluft einer Person durchströmten Bauteil angeordnet ist.

3. Vorrichtung nach den Ansprüchen 1 und 2, **dadurch gekennzeichnet, dass** der Sauerstoffsensor zur selektiven Leitung von Sauerstoff-Ionen Yttriumdotiertes Zirkonoxid als Elektrolyt zwischen zwei Elektroden sowie ein Trägerelement und ein Heizelement enthält.

4. Vorrichtung nach den Ansprüchen 1 bis 3, **dadurch gekennzeichnet, dass** der Köhlendioxid-Sensor einen Elektrolyt aus einem superschnellen Natrium-lonenleiter, zwei Elektroden, ein Trägerelement und ein Heizelement enthält.

5. Vorrichtung nach den Ansprüchen 1 bis 4, **dadurch gekennzeichnet, dass** Leistungsdaten erfasst sowie weitere medizinische Kenngrößen des Nutzers, wie beispielsweise die Herzfrequenz, aufgezeichnet werden.

6. Vorrichtung nach den Ansprüchen 1 bis 5, **dadurch gekennzeichnet, dass** sie Mittel zur zwei- und/oder dreidimensionalen visuellen Darstellung, mindestens ein akustisches Ausgabe- und/oder Aufnahmemittel und Mittel zur Erzeugung von Wind, Temperatur und/oder Geruch umfasst.

7. Vorrichtung nach den Ansprüchen 1 bis 6, **dadurch gekennzeichnet, dass** sie ein Mittel für die Stimulation des Tastsinns und/oder ein Mittel zur Veränderung der Zusammensetzung der Atemluft enthält.

8. Vorrichtung nach den Ansprüchen 1 bis 7, **dadurch gekennzeichnet, dass** die Komponenten der Trainings- und/oder Rehabilitationseinheit, des Systems zur Ab- und/oder Anreicherung von Luftbestandteilen, der Sensor-Einheit und der Steuereinheit für die Sensoren über ein Computersystem miteinander verbunden sowie gesteuert und/oder ausgelesen werden.

9. Vorrichtung nach den Ansprüchen 1 bis 8, **dadurch gekennzeichnet, dass** der Steuercomputer mit einem oder mehreren Eingabegeräten mit mindestens sechs Freiheitsgraden zur Bestimmung der Position und Orientierung verbunden ist und die Eingabegeräte wahlweise mit einer oder mehreren Tasten ausgestattet sind.

10. Vorrichtung nach den Ansprüchen 1 bis 9, **dadurch gekennzeichnet, dass** die visuelle Darstellungseinheit für die Verwendung der Vorrichtung in den Bereichen Fitness, Wellness oder Medizin ein Bild mit einem Sichtwinkel von 180° oder mehr als 180° wiedergeben kann.

11. Vorrichtung nach den Ansprüchen 1 bis 10, **dadurch gekennzeichnet, dass** die akustische Ausgabeeinheit Musikinstrumente, menschliche Stimmen, Umgebungsgeräusche wie Tierlaute, Wind, Regen, Wasserfälle, Donner und/oder Geräusche von Fahrzeugmotoren, Schüssen, Pumpen, Explosionen und/oder Erdarbeiten wiedergibt.

12. Vorrichtung nach den Ansprüchen 1 bis 11, **dadurch gekennzeichnet, dass** mit dem Computersystem ein Zellanalysegerät, vorzugsweise ein Gerät für die Durchflusszytometrie, verbunden ist.

13. Verfahren zur gleichzeitigen Überwachung, Steuerung und/oder Regelung einer Gaszusammensetzung, wobei
- eine Person eine Trainingseinheit und/oder eine Rehabilitationseinheit benutzt,
- in einer Sensor-Einheit eine Sauerstoffkonzentrationsbestimmung mit Hilfe eines beheizbaren elektrochemischen Festelektrolyt-Sensors und eine Kohlendioxid-Konzentrationsbestimmung mit Hilfe eines weiteren beheizbaren elektrochemischen Festelektrolyt-Sensors erfolgt,
- eine vom Atemflussvolumen der Person abhängige Steuerung der Heizkraft von Heizelementen der Sensoren zur Aufrechterhaltung konstanter Sensor-Temperaturen mit Hilfe eines Micro-Controllers in einer Sensor-Steuereinheit erfolgt sowie
- unter Vorgabe von bestimmbaren Sollwerten und/oder in Abhängigkeit der von der Sensor-Einheit ermittelten Atemgaszusammensetzung sowie des ermittelten Atemflussvolumens der Personen ein System zur Erzeugung der Gaszusammensetzung Luftbestandteile ab- und/oder anreichert.

14. Verwendung der Vorrichtung nach Anspruch 1 zur Steigerung der Ausdauerleistung, vorzugsweise mittels eines Höhentrainings.

15. Computerprogramm mit Programmcode zur Durchführung aller Verfahrensschritte nach Anspruch 13, wenn das Programm in einem Computer ausgeführt wird.

## Claims

1. A device for monitoring, controlling and/or regulating a gas composition, **characterized in that** the device comprises at least
- one training and/or rehabilitation unit;
- one sensor unit having a heatable electrochemical solid electrolyte sensor for oxygen concentration determination and another heatable electrochemical solid electrolyte sensor for carbon dioxide concentration determination;
- one control unit for the sensors;
- one microcontroller in the control unit for controlling the heating power of heating elements of the sensors, depending on the respiratory flow volume, for maintaining constant sensor temperatures; and
- a system for depleting and/or enriching air components for producing the gas composition according to determinable desired values and/or depending on the breathing gas compositions detected by the sensor unit and on the detected respiratory flow volume.

2. The device according to claim 1, **characterized in that** the sensor unit is directly arranged in a component through which the inspiratory and expiratory air of a person flows.

3. The device according to claims 1 and 2, **characterized in that**, for selectively conducting oxygen ions, the oxygen sensor includes yttrium-doped zirconium oxide as an electrolyte between two electrodes as well as a carrier element and a heating element.

4. The device according to claims 1 to 3, **characterized in that** the carbon dioxide sensor includes an electrolyte composed of a sodium super-ionic conductor, two electrodes, a carrier element and a heating element.

5. The device according to claims 1 to 4, **characterized in that** performance data are collected and other medical parameters of the user such as the heart frequency are recorded.

6. The device according to claims 1 to 5, **characterized in that** it comprises means for two and/or three-dimensional visual representation, at least one acoustic output and/or recording means and means for producing wind, temperature and/or odour.

7. The device according to claims 1 to 6, **characterized in that** it includes a means for stimulating the tactile sense and/or a means for changing the composition of the breathing air.

8. The device according to claims 1 to 7, **characterized in that** the components of the training and/or rehabilitation unit, of the system for depleting and/or enriching air components, of the sensor unit and of the control unit for the sensors are connected to each other as well as controlled and/or read out by way of a computer system.

9. The device according to claims 1 to 8, **characterized in that** the control computer is connected to one or several input devices having at least six degrees of freedom for determining the position and orientation and the input devices are optionally equipped with one or several keys.

10. The device according to claims 1 to 9, **characterized in that** the visual representation unit can reproduce an image having a viewing angle of 180° or more than 180° for use of the device in the fields of fitness, wellness or medicine.

11. The device according to claims 1 to 10, **characterized in that** the acoustic output unit reproduces musical instruments, human voices, ambient noise such as animal sounds, wind, rain, waterfalls, thunder and/or noise from vehicle engines, shots, pumps, explosions and/or earthworks.

12. The device according to claims 1 to 11, **characterized in that** a cell analyzing device, preferably a device for flow cytometry, is connected to the computer system.

13. A method for simultaneously monitoring, controlling and/or regulating a gas composition, wherein
- a person uses a training unit and/or a rehabilitation unit;
- in a sensor unit, an oxygen concentration determination is performed by means of a heatable electrochemical solid electrolyte sensor and a carbon dioxide concentration determination is performed by means of another heatable electrochemical solid electrolyte sensor;
- a control of the heating power of heating elements of the sensors, depending on the respiratory flow volume of a person, is performed in a sensor control unit by means of a microcontroller for maintaining constant sensor temperatures; and
- a system for producing the gas composition depletes and/or enriches air components according to determinable desired values and/or depending on the respiratory gas composition detected by the sensor unit and on the detected respiratory flow volume of the persons.

14. Use of the device according to claim 1 for increasing endurance performance, preferably by means of high-altitude training.

15. A computer program having a program code for carrying out all steps of the method according to claim 13, when the program is executed in a computer.

## Revendications

1. Dispositif pour contrôler, commander et/ou régler une composition de gaz, **caractérisé en ce que** le dispositif comporte au moins
- une unité d'entraînement et/ou de réadaptation ;
- une unité de détection comprenant un capteur à électrolyte solide électrochimique chauffant pour déterminer la concentration en oxygène et un autre capteur à électrolyte solide électrochimique chauffant pour déterminer la concentration en dioxyde de carbone ;
- une unité de commande pour les capteurs ;
- un microcontrôleur contenu dans l'unité de commande pour la commande, dépendant du volume de flux respiratoire, de la puissance de chauffage d'éléments de chauffage des capteurs pour le maintien de températures des capteurs constantes ; et
- un système d'appauvrissement et/ou enrichissement des composants de l'air pour la production de la composition de gaz selon des valeurs de consigne définissables et/ou en fonction des compositions de gaz respiratoire déterminées par l'unité de détection ainsi que du volume de flux respiratoire déterminé.

2. Dispositif selon la revendication 1, **caractérisé en ce que** l'unité de détection est disposée directement dans un composant parcouru par l'air inspiré et expiré d'une personne.

3. Dispositif selon les revendications 1 et 2, **caractérisé en ce que**, pour la conduction sélective d'ions d'oxygène, le capteur d'oxygène contient de l'oxyde de zirconium dopé à l'yttrium en tant qu'électrolyte entre deux électrodes ainsi qu'un élément porteur et un élément de chauffage.

4. Dispositif selon les revendications 1 à 3, **caractérisé en ce que** le capteur de dioxyde de carbone contient un électrolyte composé d'un conducteur superionique au sodium, deux électrodes, un élément porteur et un élément de chauffage.

5. Dispositif selon les revendications 1 à 4, **caractérisé en ce que** des donnés de puissance sont saisies et d'autres paramètres médicales de l'utilisateur tels que la fréquence cardiaque sont enregistrés.

6. Dispositif selon les revendications 1 à 5, **caractérisé en ce qu'**il comporte des moyens de représentation visuelle en deux et/ou en trois dimensions, au moins un moyen de sortie acoustique et/ou des moyens d'enregistrement et des moyens de production de vent, de température et/ou d'odeur.

7. Dispositif selon les revendications 1 à 6, **caractérisé en ce qu'**il comprend des moyens de stimulation du sens du toucher et/ou un moyen de changement de la composition de l'air respirable.

8. Dispositif selon les revendications 1 à 7, **caractérisé en ce que** les composants de l'unité d'entraînement et/ou de réadaptation, du système d'appauvrissement et/ou d'enrichissement des composants de l'air, de l'unité de détection et de l'unité de commande pour les capteurs sont reliés les uns aux autres ainsi que commandés et/ou lus par le biais d'un système informatique.

9. Dispositif selon les revendications 1 à 8, **caractérisé en ce que** l'ordinateur de commande est relié à une ou plusieurs appareils d'entrée ayant au moins six degrés de liberté pour la détermination de la position et de l'orientation et les appareils d'entrée sont équipés au choix d'une ou de plusieurs touches.

10. Dispositif selon les revendications 1 à 9, **caractérisé en ce que** l'unité de représentation visuelle peut reproduire une image ayant un angle de vision de 180° ou plus de 180° pour l'utilisation du dispositif dans les domaines du fitness, du wellness ou de la médecine.

11. Dispositif selon les revendications 1 à 10, **caractérisé en ce que** l'unité acoustique de sortie peut reproduire des instruments de musique, des voix humaines, des bruits ambiants tels que les cris d'animaux, le vent, la pluie, les cascades, le tonnerre et/ou le bruit des moteurs des véhicules, les tires, les pompes, les explosions et/ou les travaux de terrassement.

12. Dispositif selon les revendications 1 à 11, **caractérisé en ce qu'**un appareil d'analyse cellulaire, de préférence un appareil pour la cytométrie de flux, est relié au système informatique.

13. Procédé pour simultanément contrôler, commander et/ou régler une composition de gaz, dans lequel
- une personne utilise une unité d'entraînement et/ou de réadaptation ;
- dans une unité de détection, une détermination de la concentration en oxygène est réalisée au moyen d'un capteur à électrolyte solide électrochimique chauffant et une détermination de la concentration en dioxyde de carbone est réalisée au moyen d'autres capteurs à électrolyte solide électrochimique chauffant ;
- une commande, dépendante du volume de flux respiratoire d'une personne, de la puissance de chauffage d'éléments de chauffage des capteurs est réalisée à l'aide d'un microcontrôleur dans une unité de commande de capteur pour le maintien de températures des capteurs constantes ; ainsi que
- un système de production de la composition de gaz appauvrit et/ou enrichit des composants de l'air selon des valeurs de consigne définissables et/ou en fonction de la composition du gaz respiratoire déterminée par l'unité de détection ainsi que du volume de flux respiratoire des personnes.

14. Utilisation d'un dispositif selon la revendication 1 pour augmenter la puissance d'endurance, en préférence au moyen d'un entraînement en altitude.

15. Programme d'ordinateur pour réaliser toutes les étapes du procédé selon la revendication 13, lorsque le programme est exécuté dans un ordinateur.
